# EUROPÄISCHE PATENTSCHRIFT

(11) **EP 1 477 114 B1**
(45) Veröffentlichungstag und Bekanntmachung des Hinweises auf die Patenterteilung: **18.03.2009**
(21) Anmeldenummer: 03010701.5
(22) Anmeldetag: 13.05.2003
(51) Int. Cl.: A61B 5/046

(54) **Vorrichtung und Verfahren zur Erfassung von Vorhofflimmern**
Device and method for detecting atrial fibrillation
Procédé et dispositif pour la détection de la fibrillation auriculaire

(43) Veröffentlichungstag der Anmeldung: 17.11.2004
(73) Patentinhaber: GME Rechte und Beteiligungen GmbH, 98716 Geschwenda (DE)
(72) Erfinder: Schiessle, Edmund, Prof. Dipl.Phys. Dipl.Ing., 73614 Schorndorf (DE); Reinhardt, Roland, Dr.rer.nat. Dipl.Phys, 67269 Grünstadt (DE); Deeg, Hans-Jürgen, Dipl.Ing., 69502 Hemsbach (DE); Saborowski, F., Prof. Dr.med., 51427 Bergisch-Gladbach (DE)
(74) Vertreter: Strehl Schübel-Hopf & Partner

(56) Entgegenhaltungen:
- EP-A- 1 118 945
- WO-A-02/24068
- US-A- 5 738 104
- MARCIANO F ET AL: "Quantification of Poincare' maps for the evaluation of heart rate variability" COMPUTERS IN CARDIOLOGY, XX, XX, 25. September 1994 (1994-09-25), Seiten 577-580, XP002166711
- HUIKURI H V ET AL: "ABNORMALITIES IN BEAT-TO-BEAT DYNAMICS OF HEART RATE BEFORE THE SPONTANEOUS ONSET OF LIFE-THREATENING VENTRICULAR TACHYARRHYTHMIAS IN PATIENTS WITH PRIOR MYOCARDIAL INFARCTION" CIRCULATION, AMERICAN HEART ASSOCIATION, DALLAS, TX, US, Bd. 93, Nr. 10, 15. Mai 1996 (1996-05-15), Seiten 1836-1844, XP009009770 ISSN: 0009-7322
- HOGUE ET AL.: "RR Interval Dymancis Before Atrial Fibrillation In Patients After Coronary Artery Bypass Graft Surgery" CIRCULATION, Nr. 98, 4. August 1998 (1998-08-04), Seiten 429-434, XP002255942

## Beschreibung

Erworbene Erkrankungen des Herzens lassen sich einteilen in Erkrankungen des Endocards, des Myocards, des Pericards und des Reizleitungssystems.

Durch systematische Erfassung und Berücksichtigung der klassischen Risikofaktoren wie Übergewicht, Bluthochdruck oder Höhe des Cholesterinspiegels lassen sich nur knapp 60% der gefährdeten Patienten rechtzeitig erkennen.

Invasive klinische Untersuchungsmethoden, wie z.B. die Herzkathetermethode, die allein in Deutschland 500.000-mal pro Jahr durchgeführt wird und mit erheblichen Risiken verbunden ist, ermöglichen eine zuverlässige Voraussage, ob bei dem untersuchten Patienten ein aktueller Herzinfarkt oder eine andere Erkrankung des Herzens droht. Jeder tausendste Patient stirbt bei dieser Diagnosemethode.

Nicht-invasive klinische Untersuchungsmethoden, wie z.B. die Elektrokardiographie, haben keine so hohen Sicherheitsrisiken, ihr klinischer Stellenwert und die Zuverlässigkeit dieser Diagnostik sind nach dem Stand der Technik ebenfalls unbefriedigend und hängen zu stark von der zeitlichen Häufigkeit des Auftretens der einzelnen Symptome ab.

Bildgebende elektronische Verfahren, wie z.B. die Kernspintomographie, werden im Laufe der Zeit die invasive Herzkathetermethode ablösen. Bildgebende elektronische Verfahren lassen Entzündungsherde in Gefäßen durch ihre unterschiedlichen Gewebe gut erkennen und prinzipiell auch gut diagnostizieren. Diese Geräte sind aber sehr teuer in der Anschaffung und sehr kostenintensiv im diagnostischen Einsatz. Für Langzeitbeobachtungen sind sie jedoch wenig geeignet, da Patienten den längeren Aufenthalt in den engen Körperaufnahmeröhren im allgemeinen nicht tolerieren.

Biochemische Verfahren beruhen im allgemeinen auf Blutuntersuchungen nach sogenannten Biomarkern. Bei der bekanntesten Methode wird das Protein CRP gemessen, das entzündliche Prozesse im Körper anzeigt. Dieser sogenannte CRP-Test liefert einen Hinweis, um das Gesundheitsrisiko eines Patienten zumindest genauer abzuschätzen zu können.

Eine gute Möglichkeit zur Früherkennung von verschiedenen Herzschäden auf elektronischer Basis bietet das elektrophysiologische Erregungs- und Reizleitungssystem des Herzens selbst. Das Erregungs- und Reizleitungssystem manifestiert sich differenziert in den einzelnen Gewebearten durch unterschiedliche elektrische Potenzialmuster, elektrophysikalisch entstanden durch elektrische Polarisation und Depolarisation.

Neben den ventrikulären und den supraventrikulären Extrasystolen stellt das Vorhofflimmern die am häufigsten vorkommende Herzrhythmusstörung dar. Bei einer mittleren angenommenen Prävalenz des Vorhofflimmerns von 0,4 % bis 1 % der Bevölkerung ergeben sich allein in Deutschland 330.000 bis 830.000 betroffene Bürger. Unter Berücksichtigung der Tatsache, dass das Vorhofflimmern mit zunehmendem Lebensalter auch zunimmt und dass der Anteil der älteren Menschen in der Gesamtbevölkerung zunimmt, wird natürlich auch die Zahl der betroffenen Menschen stetig wachsen.

Das Vorhofflimmern ist durch elektrische Erregungswellen charakterisiert, die sich nicht synchronisiert im Vorhofmyokard ausbreiten und zu chaotischen Depolarisationsabläufen mit hämodynamisch unwirksamen Vorhofkontraktionen führen.

Aus diesen biophysikalischen Grundgesetzmäßigkeiten entwickelte sich das klinische nicht-invasive elektrophysikalische Diagnoseverfahren der Elektrokardiographie, d.h. einer kurvenmäßigen Aufzeichnung der elektrischen Erregungswellen im sogenannten Elektrokardiogramm (EKG). Aus dem EKG kann im Prinzip mit unterschiedlichen Verfahren und mit unterschiedlicher Exaktheit auf den Herzrhythmus, die Herzfrequenz, die Erregungsbildung und die Erregungsleitung geschlossen werden. Es wird zwischen Ruhe-EKG, Belastungs-EKG und Langzeit-EKG unterschieden.

Das Ruhe-EKG zeigt im Falle des Vorhofflimmerns zeitlich unregelmäßig angeordnete sogenannte QRS-Komplexe (man spricht dann von absoluter Arrhythmie), während die Grundlinie eine unregelmäßige Flimmerwellen (die sogenannten f-Wellen) unterschiedlicher Amplitude und Gestalt aufweist. In Abhängigkeit von der Anordnung der Elektroden auf dem Körper des Patienten sind die Flimmerwellen nicht in allen Messungen ausreichend sicher erkennbar, so dass die Diagnose "Vorhofflimmern" nur aus den zeitlich unregelmäßigen Folgen der QRS-Komplexe ausreichend sicher gestellt werden kann. Bei sehr schneller und sehr langsamer Kammerüberleitung kann jedoch die Ventrikelfrequenz relativ regelmäßig erscheinen, so dass möglicherweise auf "absolute Arrhythmie" fehldiagnostiziert wird.

Daher muss über einen längeren Zeitraum eine sehr genauen Auswertung der RR-Abstände der QRS-Komplexe erfolgen, um die Messabweichungen für die Frequenzbestimmung im Ruhe-EKG in zulässigen Grenzen zu halten.

Das Belastungs-EKG ist eine Testmethode zur Abschätzung der Herzfrequenz unter Ruhe- und Belastungsbedingungen. Es kann somit zur Abschätzung der biologischen Wirksamkeit von Antiarrhythmika eingesetzt werden. Nicht immer aber bedeutet eine Absenkung der Herzfrequenz auch eine Verbesserung der Herzarbeit. Das Belastungs-EKG kann also den funktionellen kardiopulmonalen Status eines Patienten mit Vorhofflimmern nicht ausreichend erfassen.

Das Langzeit-EKG ist eine Messmethode zur Erfassung und Registrierung proximal auftretenden Vorhofflimmerns. Damit besteht die Möglichkeit, sowohl spontan auftretende intermittierende Rhythmusstörungen als auch die sogenannten Triggerarrhythmien zu erfassen. Übliche Messzeiten sind ca. 24 bis 72 Stunden. Die Langzeit-Elektrokardiographie ist wohl zur Zeit die wichtigste Methode zur Erfassung und damit zur Diagnostik von Vorhofflimmern. Sie ist wertvoll bei der Diagnostik von Symptomen, die mindestens einmal pro Woche auftreten. Seltener auftretende Ereignisse lassen sich mit dieser Messmethode allerdings nicht erfassen. Da das Gerät relativ schwer ist, kann es nur begrenzt mobil eingesetzt werden, so dass die Messzeit nicht im medizinisch notwendigen Rahmen erhöht werden kann.

Dieses Problem lässt sich mit dem sogenannten Event-Recorder deutlich verringern. Dabei handelt es sich um ein EKG-Aufzeichnungsgerät das ähnlich wie das Langzeit-EKG über Elektroden am Patienten fixiert ist, aber ein wesentlich kleineres Gewicht und Bauvolumen besitzt. Der nur wenige Gramm schwere Recorder hat jedoch eine deutlich begrenzte Speicherkapazität, die eine EKG-Registrierung über nur drei Minuten erlaubt. Das hat zur Folge, dass der Patient auf einen Ereignisknopf drücken muss, wenn es zu einem klinischen Ereignis kommt, um die EKG-Speicherung zu starten. Die klinischen Erfahrungen mit dem Event-Recorder zur Erfassung von Vorhofflimmern sind daher auch sehr beschränkt. Nach einer neueren Studie konnten nur bei 68 % eines statistischen Kollektivs mit Symptomen wie z.B. sogenannten Palpitationen eine richtige EKG-Diagnose gestellt werden.

Zusammenfassend kann man sagen, dass sich die messtechnische und damit auch die diagnostische Sicherheit zu stark nach der Häufigkeit des Auftretens der entsprechenden Symptome richtet. Damit ist der klinische Stellenwert dieser nicht-invasiven Diagnostik nicht sehr groß.

Aus WO 02/24068 A1 ist ein System zur Erfassung insbesondere von Vorhofflimmern bekannt.

Bei diesem System werden die RR-Intervalle gemessen, ein Histogramm der ΔRR-Abweichungen gebildet und mit Histogrammen anderer, an Arrythmien leidender Patienten verglichen. Aus US-A-5 882 901 ist ein System bekannt das die im Oberbegriff des Anspruchs 1 angegebenen Merkmale aufweist.

Angesichts des oben beschriebenen Standes der Technik liegt der Erfindung die Aufgabe zugrunde, eine Vorrichtung zur zuverlässigen nicht-invasiven Erfassung des Vorhofflimmerns schaffen.

Diese Aufgabe wird mit der Vorrichtung nach Anspruch 1 gelöst.

Die erfindungsgemäße Vorrichtung lässt sich als leichte, batteriebetriebene, nicht-invasive, bioelektronische Messvorrichtung zur elektrophysikalischen Sensierung von bioelektrischen Herzpotenzialen mit numerischer algorithmusunterstützter elektronischer Auswertung und elektronischer Anzeige zur sicheren Diagnostik des Vorhofflimmerns zu einem sehr frühen Zeitpunkt der Erkrankung gestalten.

Ein Ausführungsbeispiel der Erfindung wird nachstehend anhand der Zeichnungen näher erläutert. Darin zeigen
- Fig. 1: ein Diagramm mit dem zeitlichen Verlauf der über Elektroden abgeleiteten Herzpotenzialen (EKG),
- Fig. 2: eine graphische Darstellung zur Erläuterung einer aus dem EKG nach Fig. 1 gebildeten dreidimensionalen Punktestruktur,
- Fig. 3: die Punktestruktur, die nach der in Fig. 2 erläuterten Methode aus dem Langzeit-EKG eines gesunden Herzens gewonnen wurde,
- Fig. 4: eine in gleicher Weise aus dem Langzeit-EKG eines an Vorhofflimmern leidenden Herzens gewonnene Punktestruktur, und
- Fig. 5: ein Blockschaltbild einer Vorrichtung zur Erfassung von Vorhofflimmern.

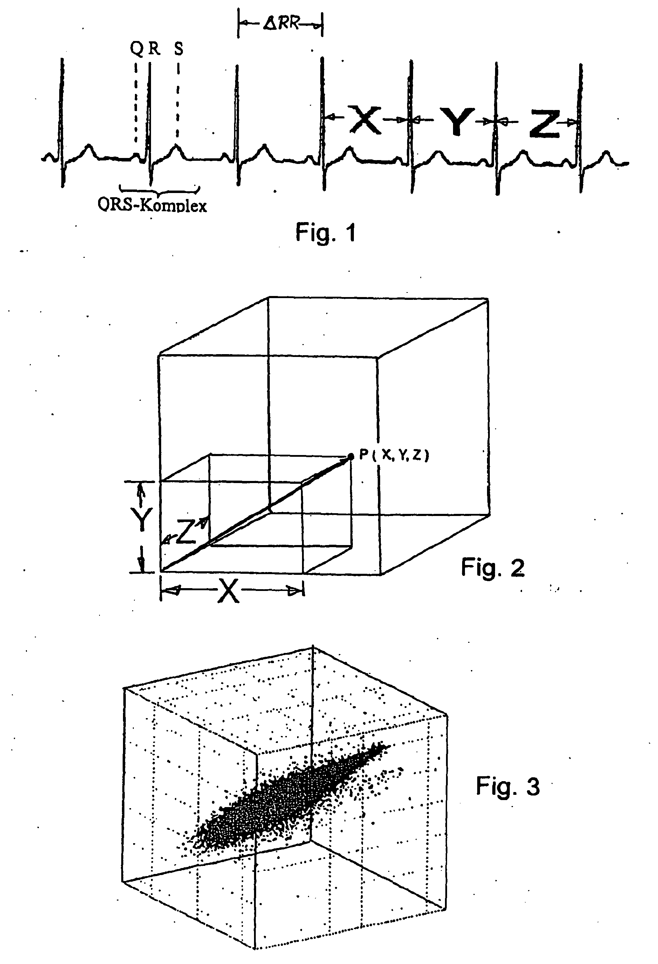

Bei der graphischen Darstellung des Langzeit-EKGs, von dem Fig. 1 einen Ausschnitt zeigt, als sogenanntem Scatter-Plot werden immer drei aufeinanderfolgende RR-Intervalle in der zeitlichen Reihenfolge der QRS-Komplexe gemessen und in einem dreidimensionalen Zahlenraum als Punktetripel Fig. 2 graphisch dargestellt, wobei die Längen der in Fig. 1 mit X, Y und Z bezeichneten RR-Intervalle an den drei Achsen des Diagramms aufgetragen werden und damit die räumliche Lage eines Punktes P ergeben. Diese Art der Darstellung ist aus dem Aufsatz von F Marciano u.a. "Quantification of Poincarè Maps for the Evaluation of Heart Rate Variability", Computers in Cardiology, 1994 IEEE, Seiten 577-580 bekannt.

Diese Auswertmethode ergibt bei einem gesunden Probanden eine dreidimensionale keulenförmige Punktstruktur, wie sie in Fig.3 veranschaulicht ist. Bei Vorliegen von Vorhofflimmern entsteht dagegen immer eine von der Keulengeometrie deutlich abweichende, gut erkennbare geometrische Punktstruktur in Gestalt einer räumlich orientierten Pyramide.

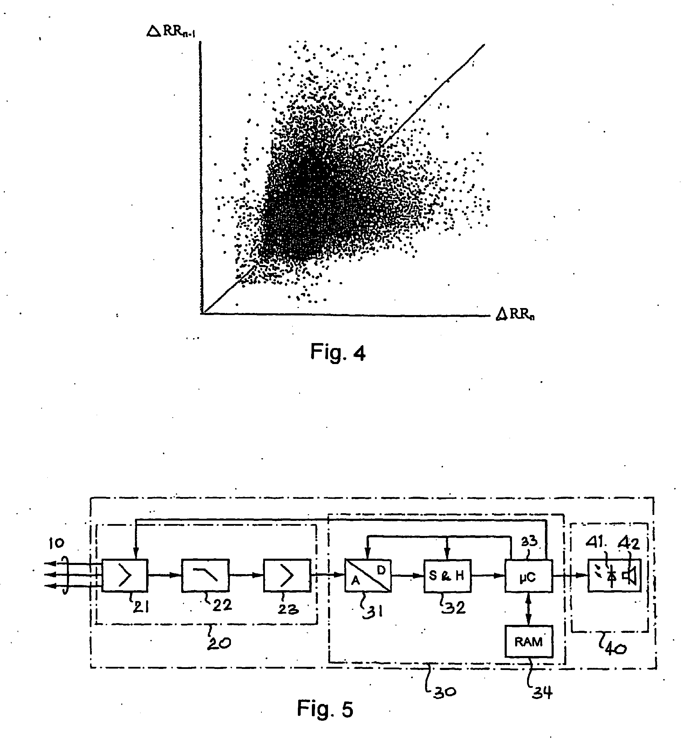

Werden jeweils nur zwei aufeinander folgende RR-Intervalle in ein zweidimensionales Diagramm eingetragen, so entsteht die in Fig. 4 gezeigte generell dreieckige Punktstruktur.

Diese biophysikalische Gesetzmäßigkeit bilden die Grundlage für den technischen Aufbau der Messvorrichtung. Aus dem EKG wird eine Liste der gemessenen RR-Intervalle gespeichert und daraus ein virtueller elektronischer, vorzugsweise zweidimensionaler Scatter-Plot erzeugt. Mit Hilfe eines vorprogrammierten speziellen Algorithmus wird der virtuelle Scatter-Plot auf das Vorhandensein einer entsprechenden Punktstruktur (in der zweidimensionalen Darstellung die Dreieckstruktur) elektronisch überprüft. Je nach dem Ergebnis der Berechnung erfolgt eine optische oder akustische Anzeige zur Erkennung des Risikos eines möglichen bevorstehenden oder schon vorhandenen Vorhofflimmerns.

Die Messvorrichtung lässt sich als leichtes handliches elektronisches Gerät bauen, das den Patienten während der Messzeit kaum belastet. Die Messungen können vom Patienten auch selbständig und unabhängig vom jeweiligen Standort ausgeführt werden. Die Anzeige des Analyseergebnisses ist einfach, so dass der Patient seinen gesundheitlichen Status sofort erkennen und im Gefahrenfalle sofort das nächste Krankenhaus oder einen entsprechenden Facharzt aufsuchen kann.

In Fig. 5 ist das elektrische Blockschaltbild der batteriebetriebenen spannungsstabilisierten technischen Ausführung der Messvorrichtung dargestellt. Sie besteht aus drei Messelektroden 10 zur Signalableitung, einem (nicht dargestellten) batteriegespeisten Konstanter zur Spannungsversorgung der elektronischen Baugruppen, einem analogen Block 20 zur analogen Signalaufbereitung, einem digitalen Block 30 zur Signalverarbeitung und einer Anzeigeeinheit 40 mit optoelektronischen und piezoelektrischen Aktoren. Der analoge Block 20 besteht aus einem hochlinearen, verstärkungsprogrammierbaren, breitbandigen Vorverstärker 21 mit sehr kleiner Temperaturdrift, einem mehrpoligen elektronischen Filter 22 und einem hochlinearen Hauptverstärker 23, der ebenfalls eine sehr kleine Temperaturtrift aufweist. Der digitale Block 30 besteht aus einem A/D-Wandler 31, einer Tastspeicherstufe (sample-and-hold-Stufe) 32, einem Mikrocontroller 33 und eine RAM-Speicher 34. Der A/D-Wandler 31 und die Tastspeicherstufe 32 sind von dem Mikrocontroller 33 taktgesteuert. Alternativ kann der A/D-Wandler in dem Mikrocontroller 33 integriert sein. Ein Signal des Mikrocontrollers 33 ist auf den Steuereingang des Vorverstärkers 21 zur Regelung der Verstärkung rückgekoppelt.

Über die drei Messelektroden 10 werden die (sehr schwachen) bioelektrischen Potenzialsignale des Herzens vom Patienten abgeleitet und über gegebenenfalls abgeschirmte Signalleitungen direkt dem Vorverstärker 21 zugeführt. Der Vorverstärker 21 generiert aus dem Potenzialsignal ein vorverstärktes elektrisches Spannungssignal. Das zwischen dem Vorverstärker 21 und dem Hauptverstärker 23 liegende Filter 22 dient dazu, Störsignalanteile von den Nutzsignalanteilen abzutrennen, eine Impulsformung durchzuführen und Antialiasingeffekte zu verhindern. Das so aufbereitete Signal wird mit Hilfe des Hauptverstärkers 23 nachverstärkt, so dass der A/D-Wandler 31 entsprechend seiner elektrischen Spezifikation angesteuert werden kann.

Der A/D-Wandler 31 erzeugt im getakteten Zusammenspiel mit dem Tastspeicherstufe 32 aus dem analogen Spannungssignal eine entsprechende digitale Bitfolge, die mit Hilfe des Mikrocontrollers 33 zeitlich geordnet in den Speicher 34 eingelesen wird. Das digitale Datenmaterial wird digital komprimiert und mit Hilfe des erwähnten Algorithmus verarbeitet, codiert und mit einer ebenfalls in dem Speicher 34 hinterlegten Referenzstruktur verglichen.

Die Messzeit kann zwischen 30 und 60 Minuten variiert werden. Nach Ablauf der gewählten Messzeit wird das Analyseergebnis über die elektronische Anzeigeeinheit 40 vorzugsweise optisch angezeigt. Da das Risiko für ein Vorhofflimmern formal in vier Stufen eingeteilt werden kann, ist die Anzeigeeinheit 40 so gestaltet, daß sie zwischen vier verschiedenen Zuständen unterscheidet. Dies kann über eine Grafik-LCD mit vier Balken oder einer in Quadranten unterteilten Kreisdarstellung geschehen. Bei der in Fig. 5 veranschaulichten Beispiel sind vier verschiedenfarbige Leuchtdioden (LEDs) 41 vorgesehen.

Leuchtet die rote LED **41**, so ist das Eintreten des Ereignisses (Vorhofflimmern) sehr wahrscheinlich. Leuchtet die gelbe LED **41**, so ist das Eintreten des Ereignisses wahrscheinlich. Leuchtet die blaue LED **41**, so ist der Eintritt des Ereignisses nicht sehr wahrscheinlich. Leuchtet die grüne LED **41**, so besteht zur Zeit überhaupt kein Risiko für das Eintreten des Ereignisses.

Um die einwandfreie Funktion des Gerätes während der Messzeit sicherzustellen ist, kann zur Zustandsüberwachung der Batterie in die Anzeigeeinheit **40** ein piezoakustischer Signalgeber **42** integriert sein, der bei zu kleiner Batteriespannung einen Pfeifton generiert und dadurch zum Austausch der Batterie vor der Messung auffordert. Alternativ kann die optische Anzeige mit einer Blinkfunktion ausgestattet sein.

Eine weitere Anwendungsmöglichkeit der hier beschriebenen Vorrichtung besteht bei einer routinemäßigen Herzuntersuchung im Rahmen einer allgemeinen Gesundheitsuntersuchung, um die am gesunden Patienten erzeugte typische keulenförmige Punktestruktur zu speichern und später als Referenzstruktur in ein Gerät einzuprogrammieren, um eine erfolgreiche Früherkennung möglich zu machen.

Ferner läßt sich die Vorrichtung bei der Früherkennung von Schlaganfällen einsetzen. Nach Untersuchung der Deutschen Stiftung für Schlaganfallhilfe und nach dem gegenwärtigen medizinischen Stand besteht eine direkte Verbindung zwischen Herzrhythmusstörungen/Vorhofflimmern und dem Auftreten eines Schlaganfalls. Nach dem Erlangener Register der Deutschen Schlaganfallhilfe beruhen 27 % aller Hirninfarkte auf Herzrhythmusstörungen.

Das Vorhofflimmern, eine Form der Herzrhythmusstörung, ist hierbei besonders gefährlich für die Entstehung eines Schlaganfalls. Dabei pumpt das Herz nur noch unregelmäßig. Weil der Vorhof lediglich Zitterbewegungen ausführt und sich nicht mehr regelmäßig zusammenzieht, wächst die Gefahr, dass sich Blutgerinnsel (Embolien) im Vorhof bilden. Wandern diese ins Gehirn, so verschließen Sie Gefäße und lösen einen Schlaganfall aus.

Die sichere Diagnostik von Vorhofflimmern zu einem möglichst frühen Zeitpunkt der Erkrankung hat einen bedeutenden Einfluss auf die Früherkennung von schlaganfallgefährdeten Personen.

## Patentansprüche

1. Vorrichtung zum Erfassen von Vorhofflimmern mit einer Äuswertschaltung **(20,30),** die aus den Herzpotenzialen des Patienten die Verteilung von RR-Intervallen berechnet, aus jeweils n aufeinanderfolgenden RR-Intervallen Punkte in einem n-dimensionalen Zahlenraum (mit n = natürliche Zahl > 1) bildet, die Struktur der n-dimensionalen Verteilung dieser Punkte in dem Zahlenraum mit der Struktur einer von einem gesunden Herz abgeleiteten normalen Verteilung vergleicht und entsprechend der Abweichung zwischen den beiden Strukturen ein den Zustand des Herzens repräsentierendes Zustandssignal erzeugt, wobei das Zustandssignal mindestens drei vom Grad der Abweichung abhängige Werte annehmen kann,
**gekennzeichnet durch** eine mit dem Zustandssignal angesteuerte optische Anzeige **(41)** mit mehreren der Anzahl der möglichen Werte des Zustandssignals entsprechenden Einzelanzeigen **(41).**

2. Vorrichtung nach Anspruch 1, wobei die optische Anzeige eine LCD mit mehreren einzeln aktivierbaren Feldern oder mehrere einzeln einschaltbare, gegebenenfalls verschiedenfarbige, LEDs **(41)** aufweist.

3. Vorrichtung nach Anspruch 1 oder 2, wobei die Auswertschaltung **(20, 30)** eine mit die Herzpotentiale ableitenden Elektroden **(10)** verbundene analoge Baugruppe **(20),** die einen Vorverstärker **(21),** ein elektronisches Filter **(22)** und einen Hauptverstärker **(23)** umfasst, sowie eine digitale Baugruppe **(30)** aufweist, die einen A/D-Wandler **(31),** eine Tastspeicherstufe **(32)** und einen Mikrocontroller **(33)** umfasst, der mit einem die Normalverteilung enthaltenden Speicher **(34)** verbunden ist.

4. Vorrichtung nach Anspruch 3, wobei der Mikrocontroller **(33)** ein auf den Vorverstärker **(21)** rückgekoppeltes, dessen Verstärkungsfaktor steuerndes Signal erzeugt.

5. Vorrichtung nach Anspruch 3 oder 4, wobei der Mikrocontroller **(33)** ein den A/D-Wandler **(31)** und die Tastspeicherstufe **(32)** steuerndes Taktsignal erzeugt.

6. Vorrichtung nach einem der vorhergehenden Ansprüche mit einer Einrichtung **(42)** zur Erzeugung eines Warnsignals bei Abfallen der Spannung einer in der Auswertschaltung **(20, 30)** vorhandenen Batterie.

7. Vorrichtung nach Anspruch 6, wobei die Einrichtung **(42)** zur Erzeugung des Warnsignals auch bei einem kritischen Wert des Zustandssignals aktivierbar ist.

8. Vorrichtung nach Anspruch 7, wobei Einrichtung **(42)** zur Erzeugung des Warnsignals eine auf Blinkfunktion umschaltbare optischen Anzeige oder einen akustischen Signalgeber aufweist.

## Claims

1. An apparatus for detecting atrial fibrillation, comprising an evaluating circuit **(20, 30)** for calculating the distribution of RR intervals from a patient's heart potentials, forming points in an n-dimensional space of numbers each from n successive RR intervals (n being a natural number > 1), comparing the structure of the n-dimensional distribution of these points in the space of numbers with the structure of a normal distribution derived from a healthy heart, and generating, in accordance with the deviation between the two structures, a state signal representing the state of the heart, which state signal is capable of assuming at least three values depending upon the degree of deviation,
**characterised by** a visual display **(41)** controlled by the state signal and including a plurality of individual displays **(41)** corresponding to the number of possible values of the state signal.

2. The apparatus of claim 1, wherein the visual display includes an LCD device having a plurality of individually activatable fields or a plurality of LCDs which can be individually turned on and which may be of different colours.

3. The apparatus of claim 1 or 2, wherein the evaluating circuit **(20, 30)** includes an analog module **(20)** connected to electrodes **(10)** deriving the heart potentials and having a preamplifier **(21),** an electronic filter **(22)** and a main amplifier **(23),** and a digital module **(30)** having an A/D converter **(31),** a sample-and-hold stage **(32)** and a microcontroller **(33)** connected to a memory **(34)** storing the normal distribution.

4. The apparatus of claim 3, wherein the microcontroller **(33)** is adapted to generate a signal which is fed back to the preamplifier **(21)** for controlling the gain thereof.

5. The apparatus of claim 3 or 4, wherein the microcontroller **(33)** generates a clock signal for controlling the A/D converter **(31)** and the sample-and-hold stage **(32).**

6. The apparatus of any preceding claim comprising means **(42)** for generating a warning signal when the voltage of a battery included in the evaluating circuit **(20, 30)** drops.

7. The apparatus of claim 9, wherein the warning signal generating means **(42)** is activatable also when the state signal assumes a critical value.

8. The apparatus of claim 7, wherein warning signal generating means **(42)** includes a visual display adapted to be switched to a flash mode or an audio signal generator.

## Revendications

1. Dispositif pour la détection de la fibrillation auriculaire avec un circuit d'interprétation (20, 30) qui calcule, à partir des potentiels cardiaques du patient, la distribution d'intervalles RR, forme à partir de n intervalles RR successifs, des points dans un espace de nombres à n dimensions (avec n = nombre naturel > 1), compare la structure de la distribution n-dimensionnelle de ces points dans l'espace de nombres à la structure d'une distribution normale dérivée d'un coeur sain, et produit, suivant l'écart entre les deux structures, un signal d'état représentant l'état du coeur, le signal d'état pouvant prendre au moins trois valeurs dépendant du degré de l'écart,
**caractérisé par** un affichage optique (41) commandé avec le signal d'état, comportant plusieurs affichages individuels (41) correspondant au nombre de valeurs possibles du signal d'état.

2. Dispositif selon la revendication 1, dans lequel l'affichage optique comporte un LCD avec plusieurs champs activables individuellement ou plusieurs LEDs (41), le cas échéant de couleurs différentes, pouvant être commandés individuellement.

3. Dispositif selon l'une quelconque des revendications 1 ou 2, dans lequel le circuit d'interprétation (20, 30) comporte un ensemble analogique (20), relié à des électrodes (10) dérivant les potentiels cardiaques, lequel comprend un préamplificateur (21), un filtre électronique (22) et un amplificateur principal (23), ainsi qu'un ensemble numérique (30) qui comprend un convertisseur analogique/numérique (31), un étage de signaux d'échantillonnage et de maintien (32) et un microcontrôleur (3) qui est relié à une mémoire (34) contenant la distribution normale.

4. Dispositif selon la revendication 3, dans lequel le microcontrôleur (33) produit un signal à rétroaction par rapport au préamplificateur (21), qui commande son facteur d'amplification.

5. Dispositif selon l'une quelconque des revendications 3 ou 4, dans lequel le microcontrôleur (33) produit un signal de rythme commandant le convertisseur analogique/numérique (31) ainsi que l'étage de signaux d'échantillonnage et de maintien (32).

6. Dispositif selon l'une quelconque des revendications 1 à 5 avec un équipement (42) pour produire un signal d'avertissement en cas de chute de la tension d'une batterie se trouvant dans le circuit d'interprétation (20, 30).

7. Dispositif selon la revendication 6, dans lequel l'équipement (42) pour produire le signal d'avertissement peut aussi être activé dans le cas d'une valeur critique de l'état de signal.

8. Dispositif selon la revendication 7, dans lequel l'équipement (42) pour produire le signal d'avertissement comporte un affichage optique commutable sur une fonction de clignotement ou un transmetteur de signaux acoustiques.
